# FASCICULE DE BREVET EUROPEEN

(11) **EP 4 076 603 B1**
(45) Date de publication et mention de la délivrance du brevet: **11.10.2023**
(21) Numéro de dépôt: 20845586.5
(22) Date de dépôt: 15.12.2020
(51) Int. Cl.: A61M 11/00, A61M 11/02, A61M 15/08, B05B 11/02, G16H 20/13, G16H 40/63, G16H 40/67, A61M 5/50

(54) **DISPOSITIF DE DISTRIBUTION DE PRODUIT FLUIDE**
VORRICHTUNG ZUR AUSGABE EINES FLUIDPRODUKTS
DEVICE FOR DISPENSING A FLUID PRODUCT

(30) Priorité: 19.12.2019 FR 1915031
(43) Date de publication de la demande: 26.10.2022
(73) Titulaire: APTAR France SAS, 27110 Le Neubourg (FR)
(72) Inventeur: BAILLIE, Mickaël, 27400 Acquigny (FR); HUPPÉ, Maxime, 76320 Caudebec les Elbeuf (FR); NORRANT, Matthieu, 27120 Houlbec Cocherel (FR)
(74) Mandataire: CAPRI
(86) Numéro de dépôt international: PCT/FR2020/052458
(87) Numéro de publication internationale: WO 2021/123613

(56) Documents cités:
- EP-A1- 0 546 607
- WO-A1-2013/154954
- WO-A1-2016/097603
- WO-A2-01/93926
- WO-A2-2008/091838
- CN-B- 102 626 533
- US-A1- 2009 128 330
- US-A1- 2016 193 408
- US-A1- 2017 257 436

## Description

La présente invention concerne un dispositif de distribution de produit fluide, et plus particulièrement un dispositif du type unidose ou bidose, adapté à distribuer une ou deux doses de produit fluide en un seul actionnement.

Les dispositifs du type unidoses ou bidoses sont bien connus. Ils comportent généralement un réservoir contenant une ou deux doses de produit fluide, et une tête de distribution pourvue d'un orifice de distribution, et qui est déplaçable axialement par rapport audit réservoir lors de l'actionnement. Les documents EP0546607 et WO2016097603 décrivent des dispositifs de ce type.

Pour certains médicaments sensibles, l'utilisation du dispositif et donc la distribution d'une dose du médicament peut indiquer une situation d'urgence. Par exemple, dans le cas des médicaments à base de Naloxone, qui sont notamment utilisés pour se remettre d'un surdosage (pour les toxicomanes mais aussi pour les utilisateurs d'opioïdes « normaux »), la durée d'activité du médicament est très limitée, d'où la nécessité de consulter rapidement un médecin ou similaire pour obtenir une autre dose une fois nécessaire. Un autre exemple concerne l'épinéphrine, où il est aussi important que le patient se rende rapidement à l'hôpital pour un suivi, car l'efficacité de l'épinéphrine n'est que de quelques heures. Il peut en être de même avec d'autres médicaments, tels que par exemple le Fentanyl.

Les documents WO2008091838, US2016193408 et WO0193926 décrivent des dispositifs qui détectent l'actionnement d'un dispositif de distribution de médicament, et qui sont aptes à émettre une alerte, par exemple vers un numéro d'appel d'urgence. Ces dispositifs permettent d'alerter lorsque le dispositif est utilisé, mais si cette utilisation a été réussie, et que la dose a été correctement distribuée, l'alerte vers un numéro d'appel d'urgence n'est en réalité pas utile. Une telle alerte est par contre hautement souhaitable si l'utilisateur tente d'actionner le dispositif, mais n'y arrive, par exemple par manque de force, perte de connaissance, ou dysfonctionnement du dispositif.

Les documents US 2017/257436 A1, CN 102 626 533 B et WO 2013/154954 A1 décrivent d'autres dispositifs de l'état de la technique.

La présente invention a pour but de fournir un dispositif de distribution de produit fluide, notamment du type unidose ou bidose, qui ne reproduit pas les inconvénients susmentionnés.

La présente invention a aussi pour but de fournir un dispositif de distribution de produit fluide, notamment du type unidose ou bidose, qui appelle automatiquement un numéro d'urgence lors d'une tentative d'actionnement du dispositif.

La présente invention a également pour but de fournir un dispositif de distribution de produit fluide, notamment du type unidose ou bidose, qui est simple et peu coûteux à fabriquer et à assembler.

La présente a donc pour objet un dispositif de distribution de produit fluide comportant un corps et une tête de distribution pourvue d'un orifice de distribution et déplaçable axialement par rapport audit corps lors de l'actionnement, ledit corps recevant un réservoir contenant une ou deux doses de produit fluide, ledit dispositif comportant un module électronique qui comprend un module de communication sans fil, tel qu'un module GSM et/ou un module Wifi et/ou un module Bluetooth^{®}, un module de localisation géographique, tel qu'un module GPS et/ou un réseau d'antennes, et une source d'alimentation, telle qu'une batterie, ledit dispositif comportant un système de capteurs pour détecter et signaler automatiquement une tentative d'actionnement dudit dispositif, ledit système de capteurs comportant au moins un premier capteur disposé sur ladite tête de distribution et/ou sur ledit corps, ledit au moins un premier capteur étant un capteur de pression, ledit système de capteurs détectant une tentative d'actionnement dudit dispositif lorsque ledit au moins un premier capteur est activé, ledit module de communication sans fil étant adapté à effectuer un appel d'urgence automatique vers un numéro d'urgence lorsque ledit système de capteurs détecte une tentative d'actionnement dudit dispositif.

Selon l'invention, ledit au moins un premier capteur est activé lorsqu'il détecte soit une force d'actionnement supérieure à 5 N, avantageusement supérieure à 10 N, soit une pression supérieure à 0,5 bar, avantageusement supérieure à 1 bar.

Selon l'invention, ledit système de capteurs comporte au moins un second capteur disposé sur ladite tête de distribution et au moins un troisième capteur disposé sur ledit corps, pour détecter une prise en main représentative d'une tentative d'utilisation dudit dispositif lorsque lesdits second et troisième capteurs sont activés simultanément.

Avantageusement, ledit au moins un second capteur est un capteur de micro courant, un capteur capacitif, un capteur infrarouge, un capteur de luminosité, un capteur audio, un capteur de température ou un capteur d'humidité.

Avantageusement, ledit au moins un troisième capteur est un capteur de micro courant, un capteur capacitif, un capteur infrarouge, un capteur de luminosité, un capteur audio, un capteur de température ou un capteur d'humidité.

Avantageusement, avant actionnement, ledit module électronique est éteint ou en mode veille, avec une consommation d'énergie nulle ou minimale.

Avantageusement, ledit module électronique est allumé ou réveillé par ledit système de capteur pour passer de son mode veille ou éteint à un mode actif.

Avantageusement, ladite tête de distribution comporte une bride radiale et ledit corps comporte une paroi axiale distale sur lesquelles l'utilisateur place ses doigts lors de l'actionnement, ledit au moins un premier capteur étant disposé sur ladite bride radiale et/ou sur ladite paroi axiale distale.

Avantageusement, ledit module électronique comporte des moyens de temporisation pour décaler l'appel d'urgence automatique de quelques secondes de la détection d'une tentative d'utilisation par le système de capteurs.

Selon une première variante avantageuse, ledit produit fluide est un liquide.

Selon une seconde variante avantageuse, ledit produit fluide est une poudre.

Avantageusement, ledit réservoir contient une seule dose de produit fluide, distribuée en un seul actionnement.

En variante, ledit réservoir contient deux doses de produit fluide, distribuées en deux actionnements successifs.

Ces caractéristiques et avantages et d'autres apparaîtront plus clairement au cours de la description détaillée suivante, faite en référence aux dessins joints, donnés à titre d'exemples non limitatifs, et sur lesquels :
la figure 1 est une vue schématique en section transversale d'un dispositif du type unidose selon un premier mode de réalisation avantageux, en position de repos, et
la figure 2 est une vue schématique en section transversale d'un dispositif du type unidose selon un second mode de réalisation avantageux, en position de repos.

Plus particulièrement, la présente invention concerne d'une part un dispositif du type unidose, tel que par exemple ceux divulgués dans les documents EP0546607 et WO02045866, et d'autre part un dispositif du type bidose, tel que par exemple celui divulgué dans le document WO2016097603.

Il est toutefois entendu que la présente invention ne se limite pas à ces types de dispositif, mais est au contraire applicable à tous les types de dispositifs de distribution de produit fluide, du type unidose ou bidose.

Dans la description, les termes "axial" et "radial" se réfèrent à l'axe longitudinale du dispositif. Les termes "proximal" et "distal" se réfère à l'orifice de distribution 3 formé dans la tête de distribution 2.

L'invention s'applique d'une part à des dispositifs du type unidose tel que ceux représentés sur les figures, dans lesquels la totalité de la dose de produit fluide contenue dans le dispositif est distribuée en un seul actionnement du dispositif, et d'autre part à des dispositifs du type bidose, dans lesquels le produit fluide contenu dans le dispositif est distribué en deux actionnements successifs du dispositif.

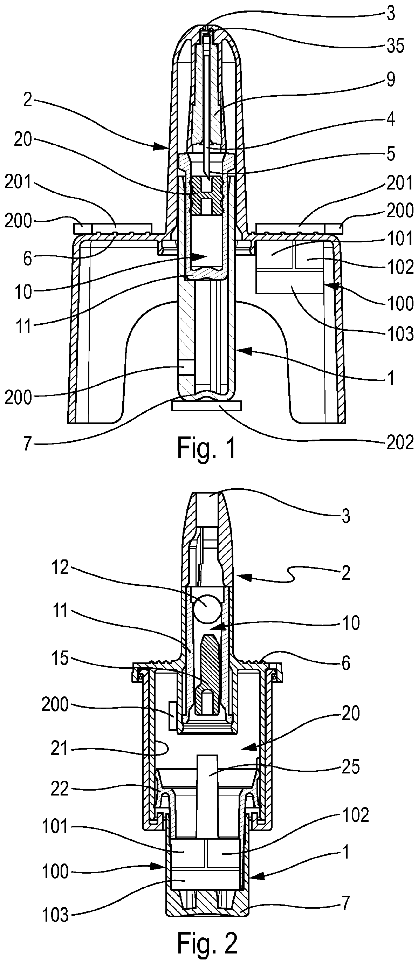

Le dispositif du type unidose représenté sur la figure 1 comporte un corps 1 qui reçoit un réservoir 10 contenant une dose de produit fluide, généralement un liquide, et une tête de distribution 2, pourvue d'un orifice de distribution 3, qui est déplaçable axialement par rapport audit corps 1 lors de l'actionnement.

Avantageusement, ledit réservoir 10 est formé par un tube creux borgne 11, par exemple en verre, ayant une ouverture axiale proximale 12 fermée par un bouchon 20, par exemple en élastomère, adapté à coulisser de manière étanche dans ledit tube 11 lors de l'actionnement.

La tête de distribution 2 comporte généralement une canule ou aiguille 4, de forme généralement cylindrique, reliée d'un côté audit orifice de distribution 3, et pourvue de l'autre côté d'une pointe de perçage 5 adaptée à percer ledit bouchon 20 lors de l'actionnement, le bouchon 20 se déplaçant alors dans ledit réservoir 10 pour expulser la ou les doses de produit fluide à travers ladite canule 4 vers ledit orifice de distribution 3. La canule 4 peut être insérée dans un support de canule 9, qui lui-même peut être fixé dans ladite tête de distribution 2. Avantageusement, un profil de pulvérisation 35 peut être formé directement en amont de l'orifice de distribution 3, par exemple entre le fond de ladite tête de distribution 2 et l'extrémité axiale proximale dudit support de canule 9.

La tête de distribution 2 comporte une bride radiale 6 sur laquelle l'utilisateur place un ou plusieurs doigts lors de l'actionnement.

La figure 2 illustre un autre type de dispositif, dans lequel le produit fluide est une poudre.

Le dispositif représenté sur la figure 2 comporte un réservoir 10 contenant une seule dose de poudre. Une tête de distribution nasale 2 est assemblée sur ledit réservoir 10, ladite tête étant destinée à être insérée dans une narine d'un utilisateur. Ladite tête de distribution nasale 2 comporte un orifice de distribution 3. La tête de distribution 2 comporte avantageusement une bride radiale 6 formant repose-doigt s'étendant radialement pour faciliter l'actionnement. Le dispositif comporte en outre une chasse d'air 20 générant, lors de l'actionnement dudit dispositif, un écoulement d'air comprimé pour distribuer une dose de poudre à travers ledit orifice de distribution 3. Ladite chasse d'air comprend une chambre d'air 21 et un piston 22 coulissant de manière étanche dans ladite chambre d'air 21 pour comprimer l'air contenu dans ladite chambre d'air 21 et ainsi générer ledit écoulement d'air comprimé. Le piston 22 est de préférence solidaire d'un corps 1 formant organe d'actionnement sur lequel l'utilisateur va appuyer lors de l'actionnement pour déplacer le piston 22 dans la chambre d'air 21. Dans l'exemple représenté sur les figures, le réservoir 10 est formé par un tube creux 11 ouvert à ses deux extrémités axiales, et fermé à son extrémité proximale par un élément de fermeture 12, tel qu'une bille, et fermé à son extrémité distale par un insert 15. Cet insert 15 comporte une extension axiale formant tige, et peut, lors de l'actionnement, coulisser dans ledit tube creux 11 pour chasser ledit élément de fermeture 12 hors de sa position de fermeture. Dans cet exemple, le piston 22 de la chasse d'air 20 est solidaire d'une projection axiale 25 qui s'étend en direction proximale, et qui, lors de l'actionnement, va se déplacer ensemble avec le piston 22 lors de la compression de l'air contenu dans la chambre d'air 21. Lorsque ladite projection 25 du piston 22 vient en contact avec ledit insert 15 du réservoir 10, une continuation du déplacement du piston 22 va provoquer le coulissement dudit insert 15 dans ledit tube creux 11 hors de sa position de fermeture. Ledit insert 15 va d'une part ouvrir le passage entre la chasse d'air 20 et le réservoir 10 et d'autre part provoquer l'expulsion de l'élément de fermeture 12. Ainsi, l'air comprimé dans la chambre d'air 21 va s'écouler dans ledit réservoir 10 et entrainer la dose de poudre hors dudit réservoir en direction dudit orifice de distribution 3. Les documents WO9946055, WO0245866, WO2015001281 et WO2017118827 décrivent des dispositifs de ce type. Bien entendu, d'autres types de dispositifs sont aussi possibles.

Typiquement, l'actionnement du dispositif dans les deux modes de réalisation représentés sur les dessins est réalisé en plaçant un ou plusieurs doigts sur ladite bride radiale 6, et un doigt, typiquement le pouce, sur une paroi axiale distale 7 du corps 1, et en déplaçant axialement le corps 1 par rapport à la tête de distribution 2.

Le dispositif comporte par ailleurs un module électronique 100 connecté à un système de capteurs 200, 201, 202.

Le module électronique 100 comporte avantageusement un module de communication sans fil 101, tel qu'un module GSM, un module de localisation géographique 102, tel qu'un module GPS, et une source d'alimentation 103, telle qu'une batterie.

Au repos, le module électronique 100 est avantageusement en mode veille, avec une consommation d'énergie minimale. En variante, il pourrait être complètement éteint au repos, avec donc une consommation énergétique nulle. Le réveil dudit module électronique est de préférence réalisé par le système de capteur.

Le système de capteurs comporte au moins un premier capteur 200 destiné à détecter une tentative d'actionnement. Ainsi, la présente invention ne détecte pas l'actionnement du dispositif, mais toute tentative d'actionnement, même infructueuse.

Ledit au moins un premier capteur 200 est un capteur de pression, notamment un capteur de force résistif (capteur FSR) dont la résistance varie en fonction de la pression qui lui est appliquée.

Dans l'exemple de la figure 1, la force d'actionnement d'un tel dispositif est typiquement de l'ordre de 40 N. Le premier capteur 200 a pour but de détecter une tentative d'actionnement, donc un effort bien inférieur à celui nécessaire à l'actionnement.

Dans l'exemple de la figure 2, la force d'actionnement d'un tel dispositif est typiquement de l'ordre de 25 N. Le premier capteur 200 a pour but de détecter une tentative d'actionnement, donc un effort bien inférieur à celui nécessaire à l'actionnement.

De préférence, ledit au moins un premier capteur 200 est activé lorsqu'il détecte une force d'actionnement supérieure à 5 N, avantageusement supérieure à 10 N. Ceci permet d'éviter de comptabiliser une simple prise en main du dispositif, sans réelle tentative d'actionnement.

De plus, dans l'exemple de la figure 2, la pression d'air dans la chambre d'air 21 lors de l'actionnement est typiquement de l'ordre de 1,5 bar.

En variante, ledit au moins un premier capteur 200 est activé lorsqu'il détecte une pression d'air supérieure à 0,5 bar, avantageusement supérieure à 1 bar. Ceci permet d'éviter de comptabiliser une simple prise en main du dispositif, sans réelle tentative d'actionnement.

Avantageusement, la détection d'une tentative d'utilisation n'est validée que si le système de capteurs détecte l'activation d'un premier capteur 200 pendant un temps minimum prédéterminé, par exemple au moins une seconde. Ceci permet d'éliminer les contacts furtifs non représentatifs d'une volonté d'utilisation du dispositif.

Dans l'exemple de la figure 1, le dispositif comporte trois premiers capteurs 200, deux sur la bride radiale 6 de la tête de distribution et un sur le corps 1. Dans cette variante, le ou les premier(s) capteur(s) 200 détecte(nt) la pression exercée par les doigts de l'utilisateur sur le dispositif lorsqu'il tente d'actionner le dispositif.

Le système de capteurs comporte en outre au moins deux autres capteurs 201, 202 destinés à détecter une prise en main de l'utilisateur représentative d'une volonté manifeste d'utilisation du dispositif. Ainsi, la présente invention détecte en plus d'une tentative l'actionnement du dispositif, une prise en main telle que réalisée lorsqu'on souhaite actionner le dispositif.

Avantageusement, le système de capteurs comporte au moins un second capteur 201, disposé sur la bride radiale 6 de la tête de distribution 2 et au moins un troisième capteur 202, disposé dans la paroi axiale distale 7 du corps 1. Ces seconds et troisièmes capteurs 201, 202 détectent les doigts que l'utilisateur pose d'une part sur ladite bride radiale 6 et d'autre part sur ladite paroi axiale distale 7 lorsqu'il souhaite actionner le dispositif.

Dans l'exemple représenté, il y a deux seconds capteurs 201, diamétralement opposés sur ladite bride radiale 6, et un seul troisième capteur 202 sur ladite paroi axiale distale 7. Lesdits second et troisième capteurs 201, 202, selon leur structure et leur fonctionnement, peuvent être fixés sur la bride radiale 6 et sur la paroi axiale distale 7, comme illustré sur la figure 1, ou inséré dans ladite bride radiale 6 et dans ladite paroi axiale distale 7.

L'association d'au moins un second capteur 201 sur la bride radiale 6 et d'au moins un troisième capteur 202 sur la paroi axiale distale 7 permet de détecter une prise en main du dispositif qui est représentative d'une tentative d'utilisation. Ainsi, si seul l'un parmi le second et le troisième capteur 201, 202 détecte la présence d'un doigt mais pas l'autre capteur, alors l'alerte n'est pas générée. Par contre, l'activation d'un seul des seconds et troisièmes capteurs permet avantageusement de "réveiller" le module électronique 100, pour le faire passer de son mode veille en mode actif.

Lorsque les second et troisième capteurs 201, 202 sont utilisés, un seul premier capteur 200 peut être suffisant pour détecter une pression lors d'une tentative d'actionnement.

Plusieurs variantes de réalisation des seconds et troisièmes capteurs du système de capteurs sont possibles.

Selon une première variante, les seconds et troisièmes capteurs 201, 202 sont des capteurs de micro courant. Dans cette première variante, chaque capteur comporte un pôle plus et un pôle moins, et un micro courant est généré lorsqu'un doigt contacte le second capteur 201 et un autre doigt contacte le troisième capteur 202.

En variante, les seconds et troisièmes capteurs 201, 202 sont des capteurs capacitifs. Dans cette seconde variante aussi, on détecte une prise en main dite "en pince", avec au moins un doigt sur le second capteur 201 de la bride radiale 6 et au moins un doigt sur le troisième capteur 202 sur la paroi axiale distale 7.

Selon encore d'autres variantes, les seconds et troisièmes capteurs 201, 202 peuvent être des capteurs infrarouges, des capteurs de luminosité, des capteurs audio, des capteurs de températures ou des capteurs d'humidité. Eventuellement, une combinaison de capteurs est possible, le second capteur 201 n'étant pas nécessairement identique au troisième capteur 202.

Dans l'exemple de la figure 2, le dispositif comporte un seul premier capteur 200, disposé dans la chambre d'air 21. Dans cette variante, le premier capteur 200 détecte la pression d'air dans la chambre d'air 21 lorsque l'utilisateur tente d'actionner le dispositif.

Lorsque le système de capteurs détecte une tentative d'actionnement, le module GPS 102 va alors établir la position géographique du dispositif et le module GSM 101 va effectuer un appel d'urgence automatique vers un numéro d'urgence, tel que par exemple le 110 en Europe, le 15 ou le 18 en France ou le 911 aux USA.

Dans une variante avantageuse, l'appel d'urgence automatique est décalé de quelques secondes de la détection d'une tentative d'actionnement par le système de capteur, par exemple par des moyens de temporisation prévus dans le module électronique 100. Cette mise en oeuvre permet de désactiver l'appel d'urgence si dans cet intervalle le dispositif est effectivement actionné par l'utilisateur. Dans cette hypothèse, le dispositif comporterait un quatrième capteur capable de détecter l'actionnement, par exemple le déplacement axial du corps 1 par rapport à la tête de distribution 2.

L'appel d'urgence automatique peut comporter la transmission d'un message préenregistré accompagné de la position géographique déterminée par le module GPS 102. L'appel peut aussi comporter un code pour identifier automatiquement le médicament disposé dans le dispositif de distribution de produit fluide. Avantageusement, il pourrait être prévu une codification pour chaque médicament sensible auquel la présente invention s'adresse plus particulièrement. L'appel peut aussi comporter une identification de l'utilisateur ainsi qu'un horodatage.

En variante, l'appel peut mettre l'utilisateur en contact avec le service d'urgence, de sorte que l'utilisateur peut directement communiquer avec ledit service d'urgence.

Selon une première variante, le module GSM 101 peut effectuer des appels d'urgence jusqu'à ce que la batterie 103 soit vide. Selon une seconde variante, le nombre d'appel peut être prédéfini. Selon une troisième variante, le dispositif ne réalise qu'un seul appel.

Après le ou les appels, le module électronique 100 peut retourner en mode veille ou s'éteindre.

Le module de communication sans fil 101 pourrait en variante comporter un module Wifi et/ou un module Bluetooth^{®}, et plus généralement tout type de moyens de communication sans fil utilisant diverses largeurs de bande et/ou fréquences.

Le module de localisation géographique 102 pourrait en variante comporter un module Galileo, et plus généralement tout type de moyens de localisation géographique. Ainsi, le module de localisation géographique pourrait ne pas utiliser de liaison satellite, mais un réseau d'antennes, par exemple via un réseau GSM ou Wifi ou similaire. Une combinaison des deux moyens de localisation, par satellite et par antenne, est aussi envisageable.

La source d'alimentation 103 pourrait en variante être réalisée sous la forme d'un accumulateur rechargeable. Par ailleurs, un condensateur, ou tout moyen similaire, peut être associé à la source d'alimentation 103 pour permettre de délivrer, en cas de besoin, un courant élevé aux différents modules décrits ci-dessus.

La présente invention a été décrite en référence à plusieurs modes de réalisation avantageux, mais il est entendu qu'un homme du métier peut y apporter toutes modifications, sans sortir du cadre de la présente invention tel que défini par les revendications annexées.

## Revendications

1. Dispositif de distribution de produit fluide comportant un corps (1) et une tête de distribution (2) pourvue d'un orifice de distribution (3) et déplaçable axialement par rapport audit corps (1) lors de l'actionnement, ledit corps (1) recevant un réservoir (10) contenant une ou deux doses de produit fluide, ledit dispositif comportant un module électronique (100) qui comprend un module de communication sans fil (101), tel qu'un module GSM et/ou un module Wifi et/ou un module Bluetooth^{®}, un module de localisation géographique (102), tel qu'un module GPS et/ou un réseau d'antennes, et une source d'alimentation (103), telle qu'une batterie, ledit dispositif comportant un système de capteurs (200, 201, 202) pour détecter et signaler automatiquement une tentative d'actionnement dudit dispositif, ledit système de capteurs comportant au moins un premier capteur (200) disposé sur ladite tête de distribution (2) et/ou sur ledit corps (1), ledit au moins un premier capteur (200) étant un capteur de pression, ledit système de capteurs détectant une tentative d'actionnement dudit dispositif lorsque ledit au moins un premier capteur (200) est activé, ledit module de communication sans fil (101) étant adapté à effectuer un appel d'urgence automatique vers un numéro d'urgence lorsque ledit système de capteurs (200, 201, 202) détecte une tentative d'actionnement dudit dispositif, **caractérisé en ce que** ledit au moins un premier capteur (200) est activé lorsqu'il détecte soit une force d'actionnement supérieure à 5 N, avantageusement supérieure à 10 N, soit une pression supérieure à 0,5 bar, avantageusement supérieure à 1 bar, et **en ce que** ledit système de capteurs comporte au moins un second capteur (201) disposé sur ladite tête de distribution (2) et au moins un troisième capteur (202) disposé sur ledit corps (1), pour détecter une prise en main représentative d'une tentative d'utilisation dudit dispositif lorsque lesdits second et troisième capteurs (201, 202) sont activés simultanément.

2. Dispositif selon la revendication 1, dans lequel ledit au moins un second capteur (201) est un capteur de micro courant, un capteur capacitif, un capteur infrarouge, un capteur de luminosité, un capteur audio, un capteur de température ou un capteur d'humidité.

3. Dispositif selon la revendication 1 ou 2, dans lequel ledit au moins un troisième capteur (202) est un capteur de micro courant, un capteur capacitif, un capteur infrarouge, un capteur de luminosité, un capteur audio, un capteur de température ou un capteur d'humidité.

4. Dispositif selon l'une quelconque des revendications précédentes, dans lequel ladite tête de distribution (2) comporte une bride radiale (6) et ledit corps (1) comporte une paroi axiale distale (7) sur lesquelles l'utilisateur place ses doigts lors de l'actionnement, ledit au moins un premier capteur (200) étant disposé sur ladite bride radiale (6) et/ou sur ladite paroi axiale distale (7).

5. Dispositif selon l'une quelconque des revendications précédentes, dans lequel ledit module électronique (100) comporte des moyens de temporisation pour décaler l'appel d'urgence automatique de quelques secondes de la détection d'une tentative d'utilisation par le système de capteurs.

6. Dispositif selon l'une quelconque des revendications précédentes, dans lequel ledit réservoir (10) contient une seule dose de produit fluide, distribuée en un seul actionnement.

7. Dispositif selon l'une quelconque des revendications 1 à 5, dans lequel ledit réservoir (10) contient deux doses de produit fluide, distribuées en deux actionnements successifs.

## Patentansprüche

1. Vorrichtung zur Abgabe eines Fluidprodukts, die einen Körper (1) und einen Abgabekopf (2) aufweist, der mit einer Abgabeöffnung (3) versehen ist und bei der Betätigung im Verhältnis zum Körper (1) axial bewegbar ist, wobei der Körper (1) einen Vorratsbehälter (10) aufnimmt, der eine oder zwei Fluidproduktdosen enthält, wobei die Vorrichtung ein elektronisches Modul (100) aufweist, das ein Drahtloskommunikationsmodul (101) wie etwa ein GSM-Modul und/oder ein Wifi-Modul und/oder ein Bluetooth^{®}-Modul, ein Modul zur geografischen Lokalisierung (102) wie etwa ein GPS-Modul und/oder ein Antennennetzwerk und eine Stromversorgungsquelle (103) wie etwa eine Batterie umfasst, wobei die Vorrichtung ein System von Sensoren (200, 201, 202) zur Detektion und automatischen Signalisierung eines Versuchs der Betätigung der Vorrichtung aufweist, wobei das System von Sensoren wenigstens einen ersten Sensor (200) aufweist, der auf dem Abgabekopf (2) und/oder auf dem Körper (1) angeordnet ist, wobei der wenigstens eine Sensor (200) ein Drucksensor ist, wobei das System von Sensoren einen Versuch der Betätigung der Vorrichtung detektiert, wenn der wenigstens eine erste Sensor (200) aktiviert wird, wobei das Drahtloskommunikationsmodul (101) geeignet ist, einen automatischen Notruf einer Notrufnummer zu tätigen, wenn das System von Sensoren (200, 201, 202) einen Versuch der Betätigung der Vorrichtung detektiert, **dadurch gekennzeichnet, dass** der wenigstens eine Sensor (200) aktiviert wird, wenn er entweder eine Betätigungskraft größer als 5 N, vorteilhafterweise größer als 10 N, oder einen Druck größer als 0,5 bar, vorteilhafterweise größer als 1 bar detektiert, und dadurch, dass das System von Sensoren wenigstens einen zweiten Sensor (201), der auf dem Abgabekopf (2) angeordnet ist, und wenigstens einen dritten Sensor (202) aufweist, der auf dem Körper (1) angeordnet ist, um ein Ergreifen zu detektieren, das für einen Versuch der Benutzung der Vorrichtung repräsentativ ist, wenn der zweite und dritte Sensor (201, 202) gleichzeitig aktiviert werden.

2. Vorrichtung nach Anspruch 1, wobei der wenigstens eine zweite Sensor (201) ein Mikrostromsensor, ein kapazitiver Sensor, ein Infrarotsensor, ein Helligkeitssensor, ein Audiosensor, ein Temperatursensor oder ein Feuchtesensor ist.

3. Vorrichtung nach Anspruch 1 oder 2, wobei der wenigstens eine dritte Sensor (202) ein Mikrostromsensor, ein kapazitiver Sensor, ein Infrarotsensor, ein Helligkeitssensor, ein Audiosensor, ein Temperatursensor oder ein Feuchtesensor ist.

4. Vorrichtung nach einem der vorhergehenden Ansprüche, wobei der Abgabekopf (2) einen Radialflansch (6) aufweist und der Körper (1) eine distale Axialwand (7) aufweist, auf die der Benutzer bei der Betätigung seine Finger legt, wobei der wenigstens eine erste Sensor (200) auf dem Radialflansch (6) und/oder auf der distalen Axialwand (7) angeordnet ist.

5. Vorrichtung nach einem der vorhergehenden Ansprüche, wobei das elektronische Modul (100) Verzögerungsmittel aufweist, um den automatischen Notruf ab der Detektion eines Versuchs der Benutzung durch das System von Sensoren um einige Sekunden zu verschieben.

6. Vorrichtung nach einem der vorhergehenden Ansprüche, wobei der Vorratsbehälter (10) eine einzelne Fluidproduktdosis enthält, die mit einer einzelnen Betätigung abgegeben wird.

7. Vorrichtung nach einem der Ansprüche 1 bis 5, wobei der Vorratsbehälter (10) zwei Fluidproduktdosen enthält, die mit zwei aufeinanderfolgenden Betätigungen abgegeben werden.

## Claims

1. Device for dispensing a fluid product comprising a body (1), and a dispensing head (2) provided with a dispensing opening (3) and axially movable with respect to said body (1) during actuation, said body (1) accommodating a reservoir (10) containing one or two doses of fluid product, said device comprising an electronic module (100) which comprises a wireless communication module (101), such as a GSM module and/or a Wifi module and/or a Bluetooth^{®} module, a geographical locating module (102), such as a GPS module and/or an antenna array, and a power source (103), such as a battery, said device comprising a sensor system (200, 201, 202) for automatically detecting and signalling an attempt to actuate said device, said sensor system comprising at least one first sensor (200) disposed on said dispensing head (2) and/or on said body (1), said at least one first sensor (200) being a pressure sensor, said sensor system detecting an attempt to actuate said device when said at least one first sensor (200) is activated, said wireless communication module (101) being adapted to make an automatic emergency call to an emergency number when said sensor system (200, 201, 202) detects an attempt to actuate said device, **characterized in that** said at least one first sensor (200) is activated when it detects either an actuating force greater than 5 N, advantageously greater than 10 N, or a pressure greater than 0.5 bar, advantageously greater than 1 bar, and **in that** said sensor system comprises at least one second sensor (201) arranged on said dispensing head (2) and at least one third sensor (202) arranged on said body (1), to detect a handling representative of an attempt to use said device when said second and third sensors (201, 202) are activated simultaneously.

2. Device according to claim 1, wherein said at least a second sensor (201) is a microcurrent sensor, a capacitive sensor, an infrared sensor, a luminosity sensor, an audio sensor, a temperature sensor or a humidity sensor.

3. Device according to claim 1 or 2, wherein said at least a third sensor (202) is a microcurrent sensor, a capacitive sensor, an infrared sensor, a luminosity sensor, an audio sensor, a temperature sensor or a humidity sensor.

4. Device according to any one of the preceding claims, wherein said dispensing head (2) comprises a radial flange (6) and said body (1) comprises a distal axial wall (7) on which the user places their fingers during actuation, said at least a first sensor (200) being arranged on said radial flange (6) and/or on said distal axial wall (7).

5. Device according to any one of preceding claims, wherein said electronic module (100) includes timeout means to postpone the automatic emergency call by a few seconds from detecting an attempt to use by the sensor system.

6. Device according to any one of preceding claims, wherein said reservoir (10) contains one single dose of fluid product, dispensed in one single actuation.

7. Device according to any one of claims 1 to 5, wherein said reservoir (10) contains two doses of fluid product, dispensed in two successive actuations
